# EUROPEAN PATENT APPLICATION

(11) **EP 2 728 351 A1**
(43) Date of publication of application: **07.05.2014**
(21) Application number: 13191066.3
(22) Date of filing: 31.10.2013
(51) Int. Cl.: G01N 33/18, G01N 17/00

(54) **Method and apparatus for monitoring the formation of deposits**

(30) Priority: 02.11.2012 NL 2009745
(71) Applicant: KWR Water B.V., 3433 PE Nieuwegein (NL)
(72) Inventor: Van Der Kooij, Dirk, 3435 VH Nieuwegein (NL); Van Der Wielen, Paul Wilhelmus Johannes Josephus, 3995 BA Houten (NL)
(74) Representative: Bras, Pieter

(57) **Abstract**

Method and apparatus for monitoring the formation of deposits, such as biofilms, on surfaces which are exposed to a flowing aqueous liquid, comprising a plurality of removable containers (9) placed in a parallel, wherein each container (9) contains sample pieces, such as glas beads.
The parallel installation allows for the continuous inspection of the formation of deposits. The containers (9) can be replaced and inspected one after another, without disturbing the growth of the deposits on the other samples.
The apparatus comprises flow meters (13) and the flow rate is adjustable.

## Description

### Introduction

The invention relates to a method and apparatus for obtaining a continuous overview of deposit or biomass accumulation on surfaces in time, in particular an overview of the formation of deposits on surfaces which are exposed to a flowing aqueous liquid. The invention is especially but not exclusively directed to monitoring the biomass accumulation rate.

When drinking water flows through a mains, a small layer of bacterial material, also called biofilm, will be formed on the inner surface of the mains, together with sediments. The formation of this biofilm in systems for the distribution of drinking water and in drinking water installations is undesirable for public health, aesthetic and technical reasons. Some types of bacteria which grow in a biofilm have pathogenic properties such as the Le*gionella pneumophila* bacterium, the causative agent of the so-called Legionnaire's disease. Also *Pseudomonas aeruginosa* and representatives of a number of other genera, e.g. *Mycobacterium,* can cause disease in immunocompromised persons. Growth of heterotrophic bacteria, bacteria belonging to the *Aeromonas* genus and coliform bacteria in biofilms and sediments can result in non-compliance of the water with the legal requirements. Aesthetic aspects include colour problems (brown water), turbidity and flavour problems. Research has shown that due to the formation of biofilm on the mains surface, the hydraulic resistance thereof can increase noticeably.

The formation of a biofilm is dependent upon the nature and the concentration of biodegradable matter ("sources of energy") in drinking water. These sources of energy include organic and also certain inorganic compounds (e.g. ammonia). There are however more factors which presumably enhance biomass accumulation. Such factors comprise the type of material of which the mains are made, e.g. cast iron, or certain plastic pipes of rubber components, which enhance microbial growth, the age of the mains material, the water temperature, the flow velocity and the occurrence of physicochemical processes such as adsorption and sedimentation.

### Prior art

In order to study the effect of certain circumstances on the accumulation of biomass on the surface and/or to study the effect of the formation of biofilm on the quality of the drinking water, use is made of so-called biofilm monitors or reactors.

WO93/01497 describes a method for monitoring the formation of deposits or growth such as biofilm on surfaces which are exposed to a liquid essentially containing water, wherein the liquid is carried only once without being circulated through a tube section, through and along a series of sample tube pieces being successively removed each time after a period of flow without being replaced and being treated to determine the biofilm formation thereon. In the apparatus used to perform this method the tube section is arranged essentially vertically, the flow through it being a downward one and each time the uppermost sample tube piece is removed.

The method and apparatus described in WO93/01497 provides a manner of determining the formation of deposits, in particular biofilm, on surfaces of all sorts of pipes.

DE 44 12 284 C1 discloses a method for the monitoring of the formation of biofilms on surfaces exposed to a water flow. In this method the water flows without circulation around sample-modules. The method of DE 44 12 284 C1 comprises providing several sample modules arranged in parallel and individually removable, directing a water flow towards each of the sample-modules, draining of the water after the flowing through the modules, removing a sample-module after a predetermined period while the other sample modules remain exposed to the flow, removing a further sample-module, analysing the samples from the sample-modules by microscopy and continuation of the monitoring. DE 44 12 284 C1 discloses that in case a sample-module upon microscopic analysis does not show any change in bioaccumulation compared to a first removed sample module after a predetermined period, the same sample-module may be put back into the apparatus for an additional time period.

Under conditions wherein biofilm formation actually does take place, which is usually the case when biofilm formation is monitored, the method disclosed in DE 44 12 284 C1 does not provide a continuous overview of the biofilm formation that can be carried out for an unlimited time period.

### Summary of the invention

The inventors have found that the monitoring of the formation of biofilm on surfaces exposed to a flowing aqueous liquid can be improved by using sample pieces, contained in containers placed in parallel fashion, dividing the aqueous liquid into separate flows and passing each flow once through a container, removing at least one container containing sample pieces after a period of exposure to the flowing aqueous liquid while not removing at least one container, continuing the flow of the aqueous liquid through said container(s) and replacing the removed container by new container(s) containing fresh sample pieces.

Therefore in one aspect the invention provides a method for monitoring the formation of deposits on surfaces as described in claim 1.

In a further aspect the invention provides an apparatus for performing the above described method, as described in claim 11.

Further preferred aspects and embodiments of the invention are described in the dependent claims.

According to the invention containers, containing sample pieces, are placed in a parallel fashion with respect to each other. The advantage of using containers placed in a parallel fashion with respect to each other is that the supply of bacteria and degradable substances does not change due to the presence of containers placed on top of the first, which would be the case when containers were mounted in series. For instance, when arranged on top of each other, a lower portion of containers would possibly receive a different amount of biomaterial of possibly different nature, because in such an arrangement, a portion of the material flowing through would be scavenged by the sample pieces in the containers in the top portion. This would lead to confusing and incomparable data with respect to the deposits formed on the sample pieces.

According to the invention, a container, containing sample pieces, will be removed at intervals after it has been exposed for a time to real circumstances, that is to say non-circular flow, in order to subject the depository film which has developed thereon to closer investigation. Withdrawing the sample pieces at intervals makes it possible to exactly determine the influence of exposure time on the accumulation of biomass and other deposits under defined and controlled conditions.

Sample pieces used in the method of the invention are placed in removable containers. This allows easy removal of the sample pieces without the risk of damaging the deposited matter. The containers are separately removable. The separate accessibility of the containers limits the interference necessary each time one of the containers is removed which is advantageous in view of the likelihood of mistakes occurring and in view of the time required for the tests.

The method of the invention has the advantage that it gives a continuous overview of the formation of deposits and especially of biomass accumulation and biofilm formation can be obtained with it. Moreover, this embodiment is in particular suitable when limited space is available, because this way the method can be performed with only a limited number of containers at a time. The cycle of removing containers with sample pieces and analysing the amount of deposit accumulation formed on these sample pieces and replacing the removed containers for new containers with fresh sample pieces can be carried out for an unlimited time period. This way a continuous overview of biofilm formation over an endless time period can be obtained. A moment to stop monitoring is therefore solely dependent on the decision of the user. The fact that new containers with fresh sample pieces are used to replace the removed containers furthermore opens the possibility to use any suitable analysis method, even if such a method damages the sample pieces, because the analysed sample pieces do not have to be placed back in the apparatus of the invention to be subjected to further deposit formation.

With the present invention the inventors developed a method of determining the accumulation of deposits, in particular biomass wherein the determination of biomass accumulation rate and biofilm formation require a relative short period of testing. Furthermore the method of the present invention only requires a relatively small volume of liquid to be tested. Third, the method of the invention enables the determination of the formation of deposits over an unlimited time span, which was not possible with the method described in WO93/01497 and DE 44 12 284 C1 as in the latter methods the number of the sample pieces is limited and the removed sample pieces cannot be replaced in case formation of deposits has occurred on the sample pieces. Furthermore, the method and apparatus of the invention provide cheap, simple and reliable means to determine the formation of deposits on surfaces, which are exposed to a flowing aqueous liquid.

### Short description of the figures

Fig. 1 is a front view of an exemplary embodiment of the apparatus of the invention.
Fig. 2 is a rear view of an exemplary embodiment of the apparatus of the invention.
Fig. 3 shows biomass accumulation rate (BAR) of water after granular activated carbon filtration and after ClO₂ dosage to this water in a period of about 185 days, with changing water temperature (line without symbols).

### Detailed description of the invention

According to the invention at least one container containing sample pieces is removed after a period of exposure to the flowing aqueous liquid and the sample pieces contained therein are analysed, while at least one container is not removed and the flow of the aqueous liquid is continued through the at least one container that is not removed for a further period of time, after which this container is also removed and the sample pieces contained therein are analysed. Furthermore, at least one of the removed containers are replaced by at least one container containing fresh sample pieces, the flow of the aqueous liquid is continued through said at least one new container for a period of time, after which said at least one container is removed for analysis of the sample pieces therein and replaced by new containers with fresh sample pieces. Each of the removed containers is replaced by a new container containing fresh sample pieces.

In an embodiment of the invention the plurality of removable containers comprises at least four containers, wherein after each period of time at least two containers containing sample pieces are removed and replaced with new containers containing fresh sample pieces. This way at least duplicate results are obtained, which consolidates the reliability of the method.

In an embodiment of the invention removal of the containers is carried out with constant time intervals between removal of the containers, wherein the abovementioned period of exposure to the flowing aqueous liquid is preferably between one day and four weeks, most preferably approximately two weeks.

The flow rate through the containers is preferably set such that the rate of flow of the aqueous liquid along the surface of the sample pieces corresponds with that flowing along the surface to be monitored for formation of deposits, such as the rate of flow in water mains. For instance, in the Netherlands the rate of flow would be preferably approximately 0.2 m/sec, but a lower rate can also be applied.

The surfaces to be monitored are preferably continuously exposed to a flowing aqueous liquid. Accordingly, preferably the containers are exposed to a continuous flow of liquid. Under certain circumstances, e.g. removal of containers, maintenance, stop of flow in the mains system, it is possible that there is no flow of liquid through the containers for a certain period. Preferably such a pause of flow is not too long and at least shorter than 4 to 12 hours, because a longer period of standstill will increase the risk of unreliable results.

The flow rate by which the aqueous liquid is directed through the containers is preferably constant. The water pressure of the water flowing through the containers preferably is between 0.5 and 1.0 bar. If necessary, the water pressure can be adjusted by means capable to increase the flow rate, such as a pump. This makes it possible to perform the method of the invention even under a variety of circumstances, for instance under low water pressures.

A pump also makes investigation of deposit formation under different flow rates possible. This way the invention can also be applied in experimental setups.

In addition it may be convenient to provide a reducing valve between the water supply and the inlets of the containers, so that pressure fluctuations in the mains supply are levelled out.

The aqueous liquid is preferably directed through the parallel placed containers in a continuous flow with a flow rate which is equal or at least essentially equal in all parallel containers. This makes it possible to compare results obtained from different containers.

To monitor the flow trough the containers one or more flow meters may be used, preferably one flow meter for each container. To control the flow trough the containers one or more valves may be used. Both valves and flow meters may be provided upstream or downstream of the containers.

In a further embodiment of the invention the outlet of each container is connected with an outlet member and the outlet members are connected with an outlet pipe.

In yet another embodiment the total flow rate can be further adjusted by means of a tube placed parallel to the containers between supply of the aqueous liquid and the outlet pipe, a so-called 'bypass' tube. To adjust the flow rate this parallel tube may be equipped with a valve, such as a needle valve. Upon closing the valve, the flow rate of the liquid flowing through the containers will then increase. The bypass tube may be applied as a means to limit the contact time of the flowing liquid in the main inlet system.

The aqueous liquid monitored according to the invention is preferably flowing water and especially drinking water. The method of the invention may be applied to water in any stage of the water treatment process to analyse formation of deposits at various stages and places in a water treatment system or water treatment plant.

The method is also suitable to analyse formation of deposits in systems in which liquids flow in for example food industry, pharmaceutical industry or other industries where water quality monitoring is needed for process control.

The present invention uses a plurality of containers containing sample pieces. A container as described herein may be in the form of a cuvette, tube, tubing section, column, pipe etc., but the shape of the container is not critical as long as it allows a sufficient flow through the sample pieces. The height and cross sectional dimensions of the containers will depend on factors as the desired flow rate and the amount, shape and dimension of the sample pieces. The inventors found that in practice, in particular when analyzing water in drinking water plants a suitable length of the containers is between 2 and 6 centimeter, for example 4 centimeter. A container preferably has a circular inner diameter although other shapes may be applicable as well. A suitable inner diameter of the containers is an inner diameter of between 0.75 and 1.25 cm.

The inlet opening of the container must allow the flow of the aqueous liquid as well as retain the sample pieces. In an embodiment of the invention the containers have a narrowing in the bottom which supports a sieve. The sample pieces are placed on top of the sieve. The mess width of the sieve should be such that the sieve does not influence or does not substantially influence the flow rate of the aqueous liquid passed along the sample pieces.

In a preferred embodiment of the invention each container is provided with a valve placed between the inlet of the container and the water supply which can be closed when a container is to be removed. Such a valve may be a ball valve. Alternatively, a plurality of containers is placed in a plurality of columns, each column containing at least one container.

A container may be made of any suitable material which does not influence formation of deposits. A convenient material for the containers is a transparent inert material. This makes visual inspection of containers possible.

It is preferable to arrange the containers and/or the tube sections or columns that contain the containers in an essentially vertical position and the flow is carried downstream through it. Due to this the effects of gravity are as far as possible distributed uniformly over the flow area and at the same time arranged in such a way that just a minimal space is taken up. This is of particular advantage if the containers (or tube sections, columns etc.) are connected to the public water mains and the flow takes place with water out of it. In this way deposit or biofilm specimens can be taken in various places, also outside laboratories, the small space usage being obviously very practical.

The sample pieces used in the invention provide a surface for attachment of biofilms or biomass accumulations. After removal of a container the sample pieces therein are analysed for deposit of biomass and/or other substances.

The shape, dimensions and amount of sample pieces effect the flow rate through the containers and also the rate of flow along the surface of the sample pieces and thus the deposit of biofilm.

In an embodiment of the invention the sample pieces are beads, preferably having a substantially spherical shape.

It has been found that beads can act as a filter means to scavenge large aggregates which normally would not attach to a surface, from the water flow running through the container. This could provide additional information about the quality of water or water pipes. It is important that the beads do not have a diameter that is too small, since that would increase the risk of clogging. One of the advantages of using beads is that in a rather small volume, a high contact surface can be obtained.

The sample pieces should preferably not react with water or substances in the water flowing through a container and also not release compounds in the water. A material that is particularly suited for investigating the formation of biofilm in flowing liquid is glass. Particular suitable sample pieces are glass beads. Glass beads are inert, easily obtainable and moreover, easy to be cleaned. Sample pieces may also be made of the same material as the conduit to be tested such as Teflon, polyethylene (PE), polyvinyl chloride (PVC), a metal such as copper or (stainless) steel. It is also possible to use charged particles as sample pieces, sample pieces with specific hydrophobic properties or beads with a rough surface in order to establish favourable conditions for attachment of micro-organisms thereto.

The inventors found that in practice, in particular when analyzing water in drinking water plants glass beads preferably have a diameter of approximately (i.e. +/- 1 mm) 2 mm or 4 mm or a diameter in between. Such glass beads cause a turbulent flow. This turbulent flow causes improved hydraulic conditions which accelerate the process of biofilm formation/ bioaccumulation, resulting in intensified transport of bacteria and growth inducing substances to the surface of the sample pieces. Because of this, only a relatively low water flow is required for monitoring biofilm formation and biomass accumulation. Furthermore, also the time period required for monitoring biofilm formation and biomass accumulation is shortened by the improved conditions. This makes the method of the invention perfectly suitable for use in test installations, in water outlets of water treatment plants and in water distribution systems and connected installation.

Glass beads with a smaller diameter than 2 mm may also be applied, provided that care is taken that the diameter is not too small, which would increase the risk of clogging.

Glass beads with a larger diameter than 4 mm may also be applied. This would involve of course a larger inner diameter of the containers. This would be especially applicable for research of water in which the conditions for biofilm formation or accumulation are more favourable compared to drinking water, such as surface water, untreated groundwater, water from an early stage of treatment, or treated waste water.

Because the sample pieces are contained in containers, the risk that the surface of the sample pieces, on which deposits, in particular biofilm, will be or have been developed, is touched, is minimalized.

It is also feasible to arrange the apparatus using sample pieces made of a variety of materials in different containers, for instance PVC, PE and copper. At the time of removal at least one, but preferably two containers containing PVC, PE and copper sample pieces respectively, are taken out and analysed, thus enabling comparison so that an impression of the (pipe) material for biofilm formation in a certain type of water is gained.

### Qualitative and quantitative determination of biofilm formation

Biofilm formation and biomass accumulation may be assessed by determining the amount of an indicator for biomass. The biomass accumulations and formation of biofilms are therefore preferably assessed by determining one or more of the parameters selected from the group of dry weight, cell numbers and the amount of substances selected from the group of ATP, carbohydrates, proteins and compounds of iron (Fe), manganese (Mn), aluminium (Al) and calcium (Ca), deposited on the sample pieces. The invention thus provides a method which makes it possible to obtain information about biomass accumulation rates and accumulation rates of certain chemical substances (e.g. compounds of Fe, Mn, Al and Ca) of the water. With the method of the invention changes and fluctuations with respect to the abovementioned parameters can be detected.

Variations in the amounts of iron (Fe), manganese (Mn), calcium (Ca) and/or ATP deposited on the sample pieces give an impression of variations in time of for instance water treatment/ purification systems. This makes it possible to detect effect of for example operational changes. That information could subsequently be used to optimize the water treatment or purification process. For instance, the method of the invention may be applied to monitor the effect of adding, removing or altering treatment processes on the biological stability of the water.

The invention may also conveniently be applied at selected locations in a distribution system. This enables obtaining important information about for example the (periodic) presence of Fe and Mn particles and biomass in water as a result of changes in water flow patterns as well as changes in water quality that affect biomass formation.

The quantity and the speed of deposit form an indication as to the water composition and/or the working of water treatment or storage and distribution with respect to the above mentioned compounds.

For analysis of biomass on surfaces of the sample pieces used in the method of the invention several methods may be applied. The person skilled in the art will understand that the following methods for analysis are merely meant as examples and that there are more methods that can be used to analyse the biomass on the surfaces of the sample pieces.

The concentration of active biomass in water and of biofilms can be determined by analysing adenosine triphosphate (ATP). ATP is present in all living organisms and is formed in the cell by oxidation of energy sources and used for synthesis of new cell material. Determination of ATP is based on a luciferin-luciferase reaction, wherein ATP is converted to adenosine diphosphate, while emitting light. Light sensitive detection methods are then used to detect the emitted light. Results of the ATP analysis can be obtained within a few minutes. The detection limit for direct analysis of water is approximately 0.5 ng ATP/l.

It is also possible to determine the total amount of bacteria deposited on the sample pieces using microscopy. For this purpose a volume of the water to be tested or a suspension obtained by ultrasonic treatment of a biofilm is filtered using a membrane filter (e.g. 0.2 µm). Subsequently the bacteria collected on the membrane are stained with for instance acridin orange and the numbers of bacteria are counted using fluorescence microscopy.

It is also possible to determine the amounts of iron and manganese deposited in the biomass/biofilm. High concentrations of iron and manganese in water lead to higher accumulation of bacteria, because negatively charged bacteria attach to the positively charged iron and manganese particles. For such a determination iron and manganese present in the samples is released and dissolved using nitric acid and treatment in a microwave. The concentration of iron and manganese may then be determined with a known technique such as Inductively Coupled Plasma Mass Spectrometry (ICP-MS). Detection limits for Fe and Mn in (drinking) water (after destruction) are 10 µg Fe/l and 0.6 µg/l Mn.

Measurements of other inorganic compounds (such as: aluminium, phosphate, silicate) as well as of organic compounds can also be carried out in order to gain an impression of the deposits which occur from out of the water onto the surface.

With the results of a set of sample pieces removed at chosen intervals the ATP content as a time function can be plotted out. The rate of biomass accumulation (BAR, pg ATP/cm² per day) can be calculated from the graph gradient.

### General description of the apparatus

The apparatus of the invention comprises a plurality of removable containers placed in a parallel fashion, wherein each container comprises a) sample pieces, b) an inlet, c) an inlet member connecting the inlet of the container with a supply of the flowing aqueous liquid, and d) an outlet for discharging the liquid from the container, wherein the inlet of a container is removably connected with the inlet member. The containers are removably connected, preferably by means of compression fittings. Preferably, the outlet of each container is connected with an outlet member and the outlet members are preferably connected with a main outlet system. The apparatus is preferably provided with a tubing section (bypass tube) parallel to the containers of the plurality of containers between the supply of the flowing aqueous liquid and the outlet pipe, for adjustment of total flow rate and for limiting the contact time of the flowing liquid in the inlet. Preferably, the outlet members and/or the tubing section comprise flow meters. Valves may be provided in the inlet members and/or outlet members and/or tubing section. The apparatus is suited to be connected to the public water mains, the flow of aqueous liquid taking place with water from said water mains or to a water treatment system, the flow of aqueous liquid taking place with water from said treatment system. Alternatively, the apparatus may be connected to a system in which liquids flow in for example food industry, pharmaceutical industry or other industries where water quality monitoring is needed for process control, the flow taking place with liquid from that system.

### Exemplary embodiment

The invention will now be explained in more detail by means of a purely exemplary embodiment of the apparatus according to the invention, and also by means of the use thereof.

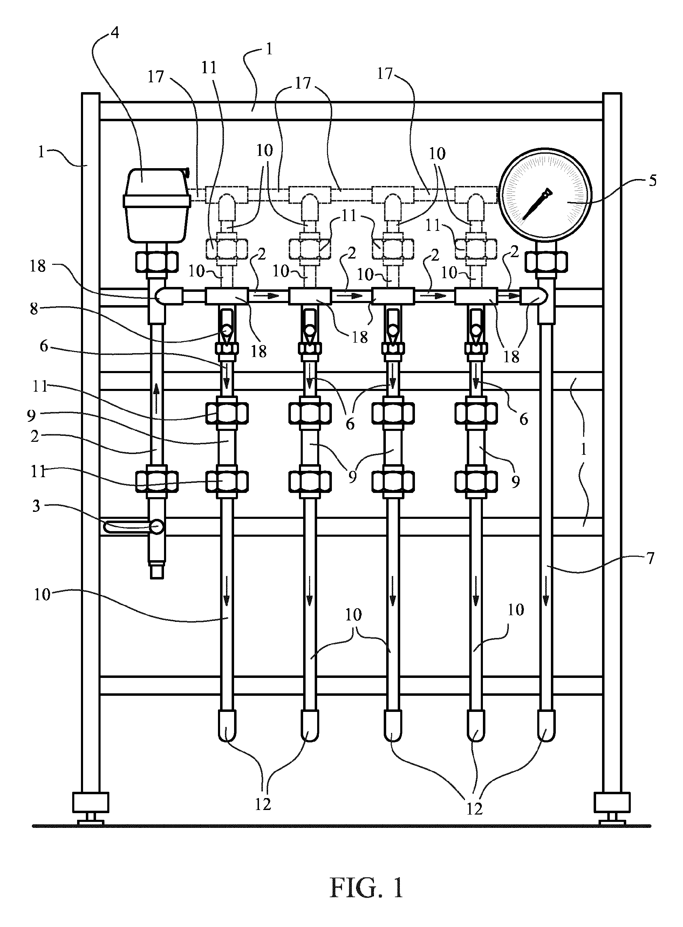

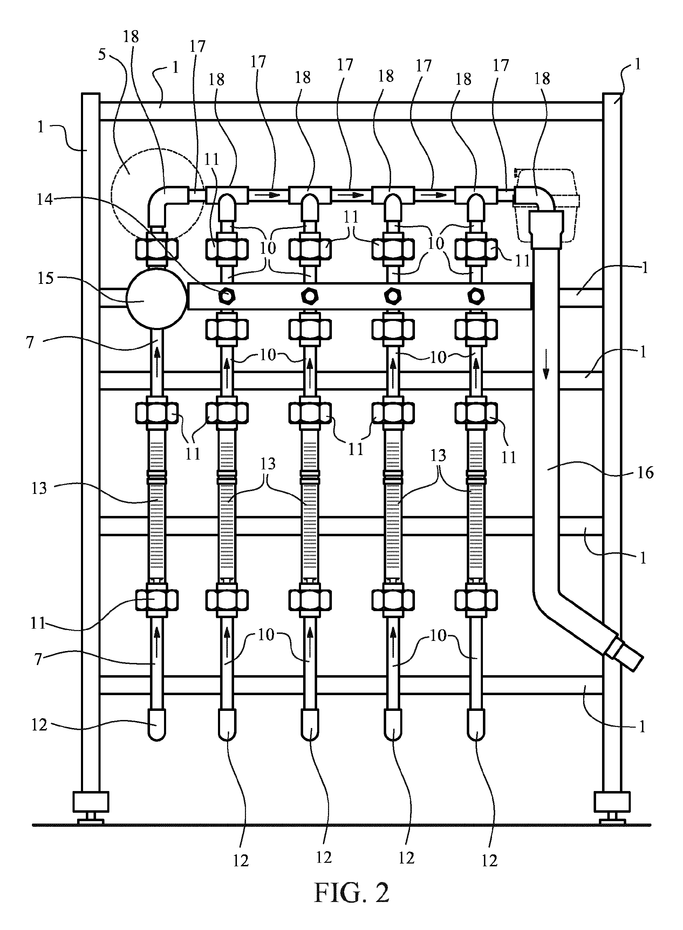

In this exemplary embodiment the biofilm monitor comprises 4 parallel placed containers with sample pieces. Each container is filled with glass beads. Fig. 1 shows a front view of an example of an apparatus of the invention and Fig. 2 shows a rear view. The direction of flowing water is indicated with arrows.

Referring now to Fig. 1 the parts of the biofilm monitor are mounted on a support 1. Water flows upwards from a water mains into a main inlet system 2, which is equipped with a ball valve 3. On top of the main inlet system a means 4 for removing air, such as air which may have been released from the water due to oversaturation or which may have entered the system during the removal of a sample piece and which might disturb a correct flow, from the system, such as an automatic air vent, and a pressure meter 5 are provided. The main inlet bends horizontally and branches off to 4 inlet members 6 and a parallel tubing section 7, which allows adjusting the flow rate of the apparatus and limiting the contact time of the flowing liquid in the main inlet system tube. The inlet members are also provided with ball valves 8 which can be closed when a container is to be removed and replaced. The containers 9 are placed between inlet members 6 and outlet members 10 by means of compression fittings 11, which enables easy coupling and decoupling of the containers. For construction purposes, where suitable connection elements 18 may be placed. On the lower side of the apparatus the outlet members make a U-turn 12 causing the water to flow upward. Also the parallel tubing section 7 makes a U-turn 12. This U-turn is provided to make the apparatus as compact as possible. As can be seen in Fig.2 the outlet members 10 and the parallel tubing section 7 are equipped with flow meters 13 which are incorporated in the outlet members by means of compression fittings 11. This way the setting of the flow rate during the experiment can be monitored while maintaining the compactness of the apparatus. Downstream of the flow meters the outlet members are equipped with a needle valve 14 for adjustment of the flow rate per container or as a closing means. Downstream of the flow meter the parallel tubing is equipped with a needle valve 15 for adjustment of the total flow rate in the apparatus. The outlet members 10 and parallel tubing 7 are connected to a main outlet system 16 which enables discharge of water from the apparatus.

The apparatus described here is compact which is of particular advantage when it is used in test installations or when it is connected to the public water mains and the flow takes place with water out of it. In this way deposit or biofilm specimens can be taken in various places, also outside laboratories, the small space usage being obviously very practical.

It is beneficial especially with respect to the desired mobility of the apparatus according to the invention, and particularly if the connection is desired at random points on the water mains supply, if the apparatus is constructed as compact and as manoeuvrable as possible, for which support means are provided for collectively supporting the component parts of the apparatus.

In a typical setup of the method of the invention, the throughput of water is 10 liter per container per hour. Every 14 days two containers are taken from the biofilm monitor for analysis and replaced by two new containers. Such an arrangement makes a continuous periodic measurement of bioaccumulation rate possible, with a constant running time of 28 days. The amount of active bacteria (active biomass) present in the containers taken from the monitor is determined using ATP analysis. Additionally, the concentration of Fe and Mn and the total amount of bacteria present on the glass beads in the container are determined.

A typical example of the application of the method of the invention in the assessment of bioaccumulation rate is described in the following example.

### Example: Assessment of the effect of chlorine dioxide dosage on the biomass accumulation rate (BAR) of treated water

The effect of the ClO₂ dosage on the biomass accumulation potential of treated water entering the distribution system was measured with the biofilm monitor as described in the exemplary embodiment described above, with four parallel containers, installed at a water treatment plant in the Netherlands and at a location in the distribution system. Water prior to ClO₂ dosage originated from granular activated carbon (GAC) filters used in the final stage of water treatment. ClO₂ dosage was applied to this water entering the storage reservoir prior to distribution. The test started at day 166 and covered a period of 185 days in which the water temperature declined due to the change of seasons.

The containers used in this example consisted of glass and were approximately 4 cm long with an inner diameter of approximately 1 cm. In the bottom of the containers there was a narrowing supporting a sieve of stainless steel. The sieve is for supporting the glass beads. On top of the sieve a ring of Teflon was mounted to secure the sieve. The mesh width of the sieve was approximately 0,2 mm with a wire diameter of approximately 0,5 mm. The container was filled with 1,68 grams of glass beads with a diameter of approximately 2 mm which results in an amount of 158 glass beads per container. The throughput of water was 10 liter per container per hour.

Every 14 days two containers were taken from the biofilm monitor for analysis and replaced by two new containers, with a constant running time of 28 days. To determine the amount of active bacteria (active biomass) present on the glass beads, the glass beads were separated from the container. The glass beads were suspended in 10 ml of sterile tap water in a tube and sonicated for 2 minutes to separate the accumulated material from the beads. The water portion was then transferred to a clean tube. The beads were supplied again with 10 ml of sterile tap water and sonicated again to separate remaining accumulated material from the beads. The water portion was then transferred to the tube with the other 10 ml of water. Subsequently the beads were supplied again with 10 ml of sterile tap water and sonicated again to separate further remaining accumulated material from the beads. The water portion was then transferred to the tube with the 20 ml of water derived from the previous sonication steps. These 30 milliliters were used for ATP determination and chemical analyses. ATP determination was analysed using a standard ATP determination procedure using a luciferin-luciferase reaction.

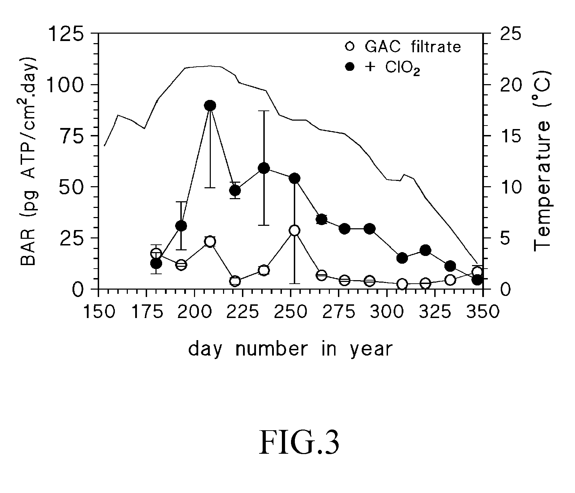

In the test period, the BAR of the granular activated carbon filtrate remained relatively low and declined with decreasing temperature (Fig. 3). The BAR value in the test system installed in the distribution system, at a location where the disinfectant residual had disappeared, attained a maximum average value of about 85 pg ATP cm⁻² day⁻¹ at a water temperature over 20°C and declined with decreasing temperature to a level of about 5 pg ATP cm⁻² day⁻¹. The elevated BAR levels, as compared to those of the granular activated carbon filtrate, are caused by the formation of easily biodegradable organic compounds due to reactions of ClO₂ with natural organic matter in the water. The BAR decline is explained by the effect of temperature on the rate of biofilm formation and the reduced level of ClO₂ dosage at decreasing temperature.

This example shows that the method and the apparatus of the invention provide convenient means to determine the formation of deposits over an unlimited period of time and that accordingly a continuous overview of formation of deposits in time can be obtained.

## Claims

1. A method for monitoring the formation of deposits on surfaces which are exposed to a flowing aqueous liquid, wherein said liquid is passed once only, without being circulated, through containers containing sample pieces, said method comprising:
a) providing a plurality of removable containers placed in a parallel fashion, each container containing sample pieces,
b) dividing the aqueous liquid to provide a flow of the aqueous liquid through each of said containers,
c) discharging the aqueous liquid after flowing through the containers,
d) removing at least one container of said plurality of containers after a period of exposure to the flow of the aqueous liquid, while not removing at least one of the containers of said plurality of containers, and continuing the flow of the aqueous liquid through the container(s) not removed,
e) removing at least one further container of said plurality of containers after a further period of exposure to the flow of the aqueous liquid, and continuing the flow of the aqueous liquid through any container(s) not removed,
f) repeating the previous step with any container(s) not removed,
g) analysing the sample pieces contained in each container for deposit formation thereon,
wherein
each of said removed containers is replaced by a new container containing fresh sample pieces, and
the flow of the aqueous liquid through said new containers is continued for a period of time after which said new containers are removed to obtain a continuous overview of deposit formation on surfaces in time.

2. The method according to claim 1, wherein said surfaces are continuously exposed to a flowing aqueous liquid.

3. The method according to claim 1 or 2, wherein the plurality of removable containers comprises at least four containers, wherein after each period of time at least two containers containing sample pieces are removed and replaced with new containers containing fresh sample pieces.

4. The method according to any of the claims 1-3, wherein the aqueous liquid is directed through the parallel placed containers in a continuous flow with a flow rate, which is equal or at least essentially equal in all parallel containers.

5. The method according to any of the claims 1-4, wherein the flow rate by which the aqueous liquid is directed through the containers is constant and adjustable by means capable to regulate the flow rate.

6. The method according to any of the claims 1-5, wherein removal of the containers is carried out with constant time intervals between removal of the containers, wherein the period of exposure to the flowing aqueous liquid is preferably between one day and four weeks, most preferably approximately two weeks.

7. The method according to any of the claims 1-6, wherein the flow rate through the containers is set such that the rate of flow of the aqueous liquid along the surface of the sample pieces corresponds with that flowing along the surface to be monitored for formation of deposits.

8. Method according to any of the claims 1-7, wherein the sample pieces are beads, preferably beads having a spherical shape, and more preferably glass beads with preferably a diameter of approximately 2 mm.

9. Method according to any of the claims 1-8, wherein the deposits are biofilms and biomass accumulations.

10. Method according to claim 9, wherein the biomass accumulations and formation of biofilms are assessed by determining one or more of the parameters selected from the group of dry weight, cell numbers and the amount of substances selected from the group of ATP, carbohydrates, proteins and compounds of iron (Fe), manganese (Mn), aluminium (Al) and calcium (Ca), deposited on the sample pieces.

11. Apparatus for monitoring the formation of deposits on surfaces which are exposed to a flowing aqueous liquid, comprising
a plurality of removable containers placed in a parallel fashion, wherein each container (9) comprises
sample pieces,
an inlet,
an outlet for discharging the liquid from the container, wherein the inlet is removably connected with an inlet member (6) for connecting the inlet of the container with a supply of the flowing aqueous liquid, each inlet member being connected to a main inlet system (2), wherein the outlet of each container is removably connected with an outlet member (10) and the outlet members are connected with an outlet pipe (17); and
a tubing section (7) parallel to the containers of the plurality of containers, provided between the main inlet system (2) and the outlet pipe (17), for adjustment of total flow rate and for limiting the contact time of the flowing liquid in the inlet, wherein said outlet members (10) and/or said tubing section (7) are equipped with flow meters (13);
wherein on the lower side of the apparatus, between the containers (9) and the flow meters (13), a U-turn (12) is provided in the outlet members (10) and the parallel tubing section (7).

12. Apparatus according to claim 11, wherein containers are removably connected by means of compression fittings (11).

13. Apparatus according to claim 12 or 13, wherein said inlet member and/or said outlet members and/or said tubing section comprise valves.

14. The method according to any of the claims 1-11, which is performed with the apparatus according to any of the claims 11-13.
